(19) **Europäisches Patentamt / European Patent Office / Office européen des brevets**

(11) **EP 4 635 412 A1**

(12) **EUROPEAN PATENT APPLICATION**

(43) Date of publication:
**22.10.2025 Bulletin 2025/43**

(21) Application number: **24170498.0**

(22) Date of filing: **16.04.2024**

(51) International Patent Classification (IPC):
**A61B 5/021** *(2006.01)* **A61B 5/022** *(2006.01)*
**A61B 5/0225** *(2006.01)*

(52) Cooperative Patent Classification (CPC):
**A61B 5/02255; A61B 5/02125; A61B 5/02225;**
A61B 2560/0223

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Koninklijke Philips N.V.**
**5656 AG Eindhoven (NL)**

(72) Inventors:
- **BOGATU, Laura Ioana**
  **5656 AG Eindhoven (NL)**
- **MUEHLSTEFF, Jens**
  **5656 AG Eindhoven (NL)**
- **SCHMITT, Lars**
  **5656 AG Eindhoven (NL)**

(74) Representative: **Philips Intellectual Property & Standards**
**High Tech Campus 52**
**5656 AG Eindhoven (NL)**

(54) **DEVICE, SYSTEM AND METHOD FOR CALIBRATING A BLOOD PRESSURE SURROGATE**

(57)     The present invention relates to a device for calibrating a blood pressure, BP, surrogate for use in monitoring a subject's blood pressure. The device comprises a BP input (51) configured to obtain time-dependent BP values during inflation of a cuff (11), a sensor input (52) configured to obtain a first time-dependent sensor signal and a second time-dependent sensor signal, and a processing unit (53) configured to compute a control signal for controlling the pressure-delivery system (10), a first calibration parameter, a second calibration parameter, and a calibration value for calibrating a BP surrogate from the first and second calibration parameters.

FIG.5

EP 4 635 412 A1

**Description**

FIELD OF THE INVENTION

The present invention relates to a device, system and method for calibrating a blood pressure, BP, surrogate for use in monitoring a subject's blood pressure.

BACKGROUND OF THE INVENTION

Arterial Blood Pressure (ABP) is a key physiological parameter relevant for medical diagnosis, prevention as well as therapy guidance. Invasive measurements - the Gold standard - provide the most accurate continuous measurements, but they can be done by trained medical staff only, and they are applied mostly in high acuity settings, which require real-time alarming and very close monitoring. Most non-invasive blood pressure measurements (NIBP) in patient monitoring are being implemented by standard cuff-based ABP measurements using automated oscillometry providing intermittent measurements (e.g. typically only every 15 min in ICU). There is an unmet need in continuous non-invasive blood pressure measurement technologies.

PAT (pulse arrival time) / PTT (pulse transit time) based BP surrogates may potentially enable beat-to-beat tracking of BP and BP changes. However, it is usually not known what the patient-specific relationship between a measured x ms change in PAT and its corresponding y mmHg change in BP is. The PAT-BP calibration is thus difficult to obtain.

SUMMARY OF THE INVENTION

It is an object of the present invention to enable and improve the calibration of a BP surrogate for use in monitoring a subject's blood pressure.

In a first aspect of the present invention a device for calibrating a blood pressure, BP, surrogate for use in monitoring a subject's blood pressure is presented comprising:

a BP input configured to obtain time-dependent BP values during inflation of a cuff of a pressure-delivery system attached at a cuff site to a subject's extremity, wherein the time-dependent BP values are measured at a BP measurement site of the subject's extremity distal from the cuff site;

a sensor input configured to obtain a first time-dependent sensor signal and a second time-dependent sensor signal, both related to the subject's heartbeat and measured during the inflation of the cuff, the first time-dependent sensor signal measured at a first sensor site of the subject's body and the second time-dependent sensor signal measured at a second sensor site of the subject's extremity distal from the cuff site and the BP measurement site; and

a processing unit configured to compute

- a control signal for controlling the pressure-delivery system to inflate its cuff up to a cuff pressure that causes the brachial vein of the subject's extremity to collapse;
- a first calibration parameter as a ratio between a maximum mean arterial pressure, MAP, change and a maximum PTT change;
- a second calibration parameter as the absolute value of pulse arrival time, PAT, derived from the first and second time-dependent sensor signals measured after the cuff inflation; and
- a calibration value for calibrating a BP surrogate from the first and second calibration parameters.

In a further aspect of the present invention a system for calibrating a BP surrogate for use in monitoring a subject's blood pressure is presented comprising:

a pressure-delivery system comprising a cuff configured to be attached at a cuff site to a subject's extremity and to deliver a pressure to the subject's extremity by inflating the cuff;

a BP pressure sensor configured to acquire time-dependent BP values at a BP measurement site of the subject's extremity distal from the cuff site during inflation of the cuff up to a cuff pressure that causes the brachial vein of the subject's extremity to collapse;

a first sensor configured be attached to a first sensor site of the subject's body and to acquire a first time-dependent sensor signal related to the subject's heartbeat during the inflation of the cuff;

a second sensor configured be attached to a second sensor site of the subject's extremity and to acquire a second time-dependent sensor signal related to the subject's heartbeat during the inflation of the cuff distal from the cuff site and the BP measurement site; and

a device as disclosed herein for computing a calibration value for calibrating a BP surrogate from the time-dependent BP values and the first and second time-dependent sensor signals.

In yet further aspects of the present invention, there are provided a corresponding method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, system, computer program and medium have similar and/or identical preferred embodiments as the claimed device, in particular as defined in the dependent claims and as disclosed herein.

The present invention presents a solution for estimating BP surrogate calibration. A standard BP cuff placed on the upper arm is inflated to a pressure up to a cuff pressure that causes the brachial vein of the subject's extremity to collapse, e.g., to a pressure larger than ~30 mmHg so that the vein at brachial site collapses. Consequently, outflow of blood from the limb is stopped, while inflow of blood continues via the artery. As a result, pressure in the distal artery increases. The change in PTT as result of the controlled change in internal arterial pressure is representative of the PTT-BP calibration.

A clinical study reveals that the method achieves good performance. Changes occurring in the vasculature distal from the cuff during cuff inflation are indicative of the PAT-BP calibration. Results indicate significantly improved performance in detection of BP changes when compared to standard, intermittent NIBP. The study also provides insights on implementation of the method in standard practice and how to combine the distal vasculature response to the controlled change in distal BP with other indexes (such as the absolute value of PAT) to further increase the accuracy of the BP surrogate calibration.

The obtained time-dependent BP values may be directly measured BP values, e.g., non-invasively or invasively measured BP values, such as MAP values, but may also be BP values computed from other BP values, e.g., may be MAP values that are computed from systolic BP (SBP) and diastolic BP (DBP) values. If a waveform is available, MAP may be derived as time-averaging of one or several heart cycles.

According to an embodiment, the processor is configured to compute the maximum MAP change in distal MAP as a difference between a maximum MAP value during the cuff inflation and the MAP value at the beginning of the cuff inflation, the MAP values being obtained from the obtained time-dependent BP values. For instance, the MAP values may be obtained from SBP and DBP values.

In another embodiment, the processor is configured to compute the maximum PTT change in distal pulse transit time, PTT, as a time interval between a pulse detected at the BP measurement site and the pulse detected at the second sensor site. For instance, PTT is continuously observed as a time difference of the two signals during cuff inflation. The process is dynamic and results in a maximum point.

The processor may be configured to compute the calibration value by setting the calibration value to a first calibration value if:

- the first calibration parameter is smaller than and outside a first parameter range from a first parameter threshold and the second calibration parameter is smaller than and outside a second parameter range from a second parameter threshold; or
- the second calibration parameter is within the second parameter range from the second parameter threshold and the first calibration parameter is smaller than and outside the first parameter range from the first parameter threshold; or
- the first calibration parameter is within the first parameter range from the first parameter threshold and the second calibration parameter is smaller than and outside the second parameter range from the parameter threshold; or
- the first calibration parameter is within the first parameter range from the first parameter threshold, the second calibration parameter is within the second parameter range from the second parameter threshold, a mean of a plurality of last first calibration parameters is smaller than the first

parameter threshold and a mean of a plurality of last second calibration parameters is smaller than the second parameter threshold.

The processor may further be configured to compute the calibration value by setting the calibration value to a second calibration value if:

- the first calibration parameter is larger than and outside a first parameter range from a first parameter threshold and the second calibration parameter is larger than and outside a second parameter range from a second parameter threshold; or
- the second calibration parameter is within the second parameter range from the second parameter threshold and the first calibration parameter is larger than and outside the first parameter range from the first parameter threshold; or

- the first calibration parameter is within the first parameter range from the first parameter threshold and the second calibration parameter is larger than and outside the second parameter range from the parameter threshold; or

- the first calibration parameter is within the first parameter range from the first parameter threshold, the second calibration parameter is within the second parameter range from the second parameter threshold, a mean of a plurality of last first calibration parameters is larger than the first parameter threshold and a mean of a plurality of last second calibration parameters is larger than the second parameter threshold.

One or more of the first calibration value, the second calibration value, the first parameter threshold, the second parameter threshold, the first parameter range and the second parameter range are generally predetermined values. In an embodiment, one or more of these parameters may be dynamic, may be changed during the measurements, or may be updated by the measurements in some way. In another embodiment, one or more of these parameters may be personalized, e.g. may be set depending on the type of patient (e.g. gender, age, weight, etc.).

Reasonable values for these parameters may be as follows: the first calibration value is in the range of -1.5 to -2.5 mmHg/ms, in particular a value of -1.8 mmHg/ms ± 10%, and/or the second calibration value is in the range of -1 to -0.5 mmHg/ms, in particular a value of -0.8 mmHg/ms ± 10%, and/or the first parameter threshold is in the range of -1 to -0.5 mmHg/ms, in particular a value of -0.7 mmHg/ms ± 10%, and/or the second parameter threshold is in the range of -0.4 to -0.2 mmHg/ms, in particular a value of -0.31 mmHg/ms ± 10%, and/or the first parameter range is in the range of 2.5 % to 7.5 %, in particular a value of 5 %, and/or the second parameter range is in the range of 2.5 % to 7.5 %, in particular a value of 5 %.

According to another embodiment, the processor is configured to compute a control signal for controlling the pressure-delivery system to inflate its cuff up to a cuff pressure equal to or larger than 25 mmHg, in particular larger than 30 mmHg or in the range of 25 to 35 mmHg. This value of the cuff pressure is preferably predetermined but may also be dynamic and changeable during the measurements or may be updated by the measurements. In an embodiment the value of this cuff pressure may be personalized / depending on the type of patient (e.g. gender, age, weight, etc.).

According to a preferred embodiment, the first time-dependent sensor signal is an ECG signal and/or the second time-dependent sensor signal is a photoplethysmography, PPG, signal, in particular a contact PPG signal or a remote PPG signal. Other options for the first time-dependent sensor signal are an ear PPG signal or a heart sound signal; further alterative embodiments are possible.

The processing unit may further be configured to determine BP values, in particular systolic BP values, by use of the computed calibration value and the first and second time-dependent sensor signals measured when no pressure is delivered to the subject's extremity. Thus, instead of measuring BP, BP values may be inferred from BP surrogates using the determined calibration.

The processing unit may particularly be configured to determine a BP value, in particular a systolic BP value, by multiplying computed calibration value with an absolute PAT value derived from the first and second time-dependent sensor signals and adding the result of the multiplication to a reference BP value, in particular a reference systolic BP value.

BRIEF DESCRIPTION OF THE DRAWINGS

These and other aspects of the invention will be apparent from and elucidated with reference to the embodiment(s) described hereinafter. In the following drawings:

Fig. 1 shows diagrams of the relationship between arterial transmural pressure and arterial volume.
Fig. 2 shows diagrams of the relationship between arterial volume and transmural pressure and corresponding arterial compliance, pulse wave velocity and PAT.
Fig. 3 shows a schematic diagram of an embodiment of a system according to the present invention.
Fig. 4 shows a schematic diagram of an embodiment of a device according to the present invention.
Fig. 5 shows a schematic diagram of an embodiment of a method according to the present invention.
Fig. 6 shows an exemplary implementation of a system according to the present invention.
Fig. 7 shows a diagram illustrating an example of different signals used in an embodiment.
Fig. 8 shows, per inflation number, a diagram of example Feature 1 values corresponding to two patients as calculated during each cuff inflation.
Fig. 9 shows a diagram of example Feature 1 values acquired from multiple patients as measured during cuff inflation.
Fig. 10 shows a diagram of Feature 2 values as measured throughout the procedure for two patients.
Fig. 11 shows a diagram of example values of Feature 2 values for nine patients.
Fig. 12 shows a diagram of data corresponding to a tenth patient that is added to the plot shown in Fig. 11.
Fig. 13 shows a diagram of an invasive arterial line signal, a PPG signal and a cuff pressure signal.

Figs. 14A and 14B show diagram illustrating various signals as obtained by a simulation.

DETAILED DESCRIPTION OF EMBODIMENTS

An exemplary calibration of between a BP surrogate and BP may be defined as:

$$calibration = \frac{dBP}{dBP_{surrogate}}, \qquad (1)$$

wherein $BP_{surrogate}$ may, e.g., be PAT, as calculated based on synchronized ECG and PPG signals and BP can be e. g. systolic pressure. Recently proposed solutions, as disclosed in WO 2023/072842 A1 and WO 2023/072730 A1 for measurement of calibration (Eq. (1)) make use of the intermittent cuff inflation to induce a controlled change in brachial transmural pressure. Transmural pressure ($P_{tm}$) is defined as:

$$Ptm = Part - Pcuff, \qquad (2)$$

Where $P_{art}$ is internal arterial pressure, $P_{cuff}$ is cuff pressure.

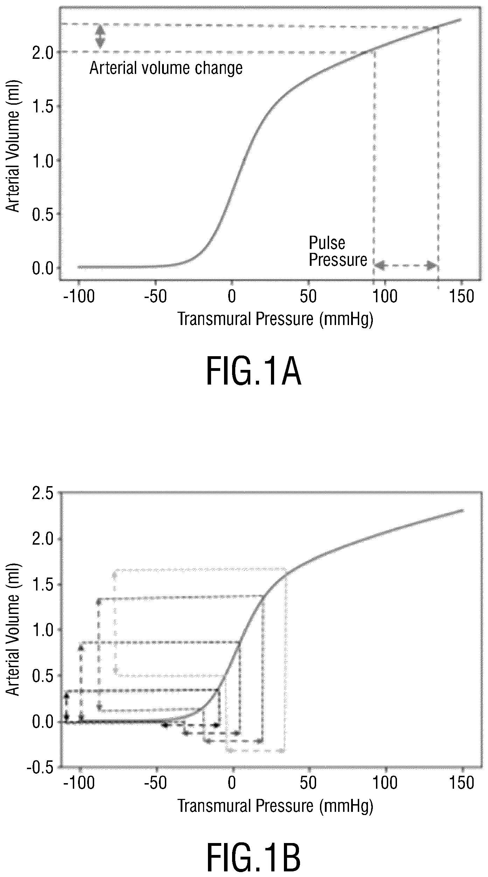

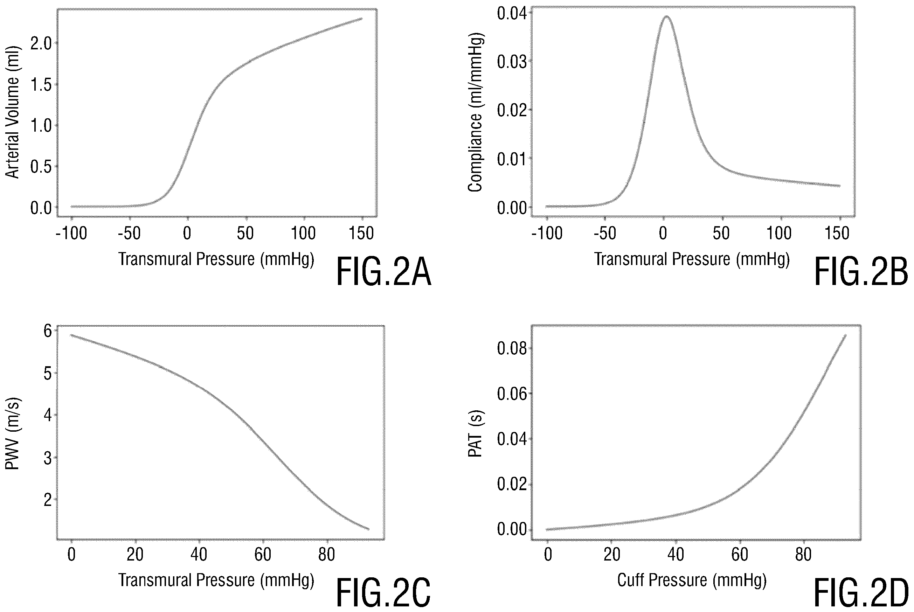

Therefore, a controlled change in $P_{cuff}$ results in a controlled change in $P_{tm}$. Fig. 1 shows diagrams of the relationship between arterial transmural pressure and arterial volume indicating arterial volume changes under a fully deflated cuff (Fig. 1A) and arterial volume oscillations (Fig. 1B), which show that the oscillations change in amplitude as the cuff exerts pressure over the arterial wall. Fig. 2 shows diagrams of the relationship between arterial volume and transmural pressure (Fig. 2A) and corresponding arterial compliance (Fig. 2B), pulse wave velocity (PWV; Fig. 2C) and PAT (Fig. 2D). Given the arterial volume - transmural pressure relationship shown in Figs. 1 and 2, an increase in $P_{cuff}$ leads to a decrease in $P_{tm}$. At lower $P_{tm}$ ranges, the artery is more compliant. The larger arterial compliance leads to a PWV decrease and respective PAT increase over the length of the cuff. The change in PAT is measured via, e.g., an ECG-PPG combination and is linked to the controlled $P_{cuff}$ change to obtain the PAT-BP relationship.

There are certain measurement aspects that require caution and careful interpretation of the cuff-induced vasculature changes at brachial site. For example, the length over which the PWV is altered is small, usually in the order of 14 cm (typical cuff length). Due to this, careful processing of the ECG-PPG signals is required due to low signal-to-noise ratio of the resulting PAT. This is particularly difficult in presence of breathing or motion artefacts. Further, the method relies on accurate control of transmural pressure, which may be difficult to achieve in some cases, because arm tissue characteristics are subject dependent. Also, in some patients, arteries are situated deep within subcutaneous fat. As a result, cuff pressure may differ from pressure at the exterior of the artery. Even in the case of accurate control of transmural pressure at centre of the cuff, there is uncertainty whether the same transmural pressure is applied along the entire length of the cuff. Ideally, such method may be complemented by additional calibration techniques to minimize such measurement uncertainties.

The inventors used multiple sensors to achieve improved understanding of cuff-induced vasculature changes. The data indicates that an alternative method may enable accurate BP surrogate calibration. The method relies on inflating the cuff to a pressure which leads to venous collapse (e.g. $P_{cuff} >\sim30mmHg$), therefore stopping outflow of blood from the limb. The cuff pressure however is kept at a low enough value to allow inflow of blood towards the limb ($P_{cuff} <\sim MAP$). Blood accumulates in the distal limb circulation with every beat, and as a result, arterial pressure increases. The distal arterial compliance decreases due to the increase in transmural pressure, PWV increases and PAT decreases. The magnitude of the PAT change is then interpreted to obtain the PAT-BP calibration.

This invention presents a method for BP surrogate calibration. The method focuses on interpreting cuff-induced changes in transmural pressure occurring distally from the cuff (as opposed to alternative methods which focus on changes occurring at brachial site). The vascular alterations occur as a result to changes in internal arterial pressure, as opposed to existing methods which rely on external cuff pressure changes. The method is therefore affected by different sources of noise. Identifying the common information from brachial/distal responses may improve the reliability of the calibration.

For example, previously proposed methods rely on accurate control of transmural pressure at the brachial site, which may be difficult to achieve in some cases due to subject-dependent arm tissue characteristics. Further, transmural pressure is non-uniform along the length of the cuff. The method according to the present invention does not depend on accurate transmural pressure modulation along the length of the cuff; the cuff is simply used to achieve complete venous collapse, e.g., by setting the cuff pressure to a value larger than ~30 mmHg.

The distal segment may be twice the length of the length of the brachial segment, potentially enabling observation of larger magnitude PAT changes for a given transmural pressure change. Another aspect is that the distal calibration method involves interpretation of vasculature response to an increase in transmural pressure. The brachial calibration method involves interpretation of vasculature response to a decrease in transmural pressure. Therefore, the two methods combined may increase the sampled transmural pressure range by revealing a BP surrogate response to a BP increase as well as a BP decrease.

The method according to the present invention alters the internal arterial blood pressure via the cuff over a well-defined arterial segment. The PAT change as response to the BP change is representative of the patient-specific BP surrogate calibration. This method allows for blood pressure changes to be achieved without impact or overlay by heart rate changes. The subject is at rest during the measurement in contrast to alternative calibration methods.

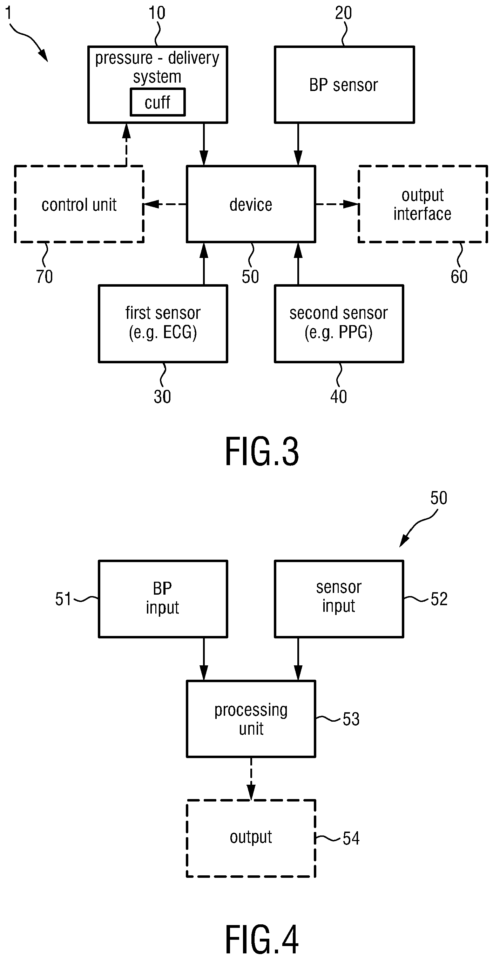

Fig. 3 shows a diagram of an embodiment of a system 1 for calibrating a BP surrogate for use in monitoring a subject's blood pressure according to the present invention.

The system 1 comprises a pressure-delivery system 10 comprising a cuff 11 configured to be at a cuff site to a subject's extremity and to deliver a pressure to the subject's extremity by inflating the cuff. Such a pressure-delivery system 10 comprising a cuff 11 that can be attached to an extremity of the subject, in particular the upper arm, upper leg or wrist, is generally known in the art of NIBP measurement. Generally, it comprises a pressure generating unit, e.g. a pump or pressure reservoir, that is configured to inflate the cuff 11, a valve that is configured to deflate the cuff 11, and a processor that is configured to control the pressure generating unit and the valve and to determine the subject's blood pressure based on the measured cuff pressure. A user interface may be provided, e.g. comprising one or more of a display, keypad, loudspeaker and touchpad, to issue the measured BP values, e.g. in visible and/or audible form.

The system 1 further comprises a pressure sensor 20 configured to acquire time-dependent BP values at a BP measurement site (e.g. the lower arm) of the subject's extremity distal from the cuff site (e.g. the upper arm) during inflation of the cuff up to a cuff pressure that causes the brachial vein of the subject's extremity to collapse. The pressure sensor 20, e.g. in the form of a pressure transducer, is preferably a device separate from the cuff and is not integrated into the cuff. It may be implemented by a conventional BP sensor. Other means to apply an external pressure and to detect cuff pressure, such as a Shellcuff design (as e.g. disclosed in US 11317819 B2 or other publications of the application regarding the Shellcuff), are possible as well.

The system 1 further comprises a first sensor 30 configured to be attached to a first sensor site of the subject's body and to acquire a first time-dependent sensor signal related to the subject's heartbeat during the inflation of the cuff. The first sensor 30 may be an ECG sensor for acquiring an ECG signal. The first sensor site may e.g. be the subject's chest or torso at which ECG electrodes are preferably attached for ECG measurements.

The system 1 further comprises a second sensor 40 configured to be attached to a second sensor site of the subject's extremity and to acquire a second time-dependent sensor signal related to the subject's heartbeat during the inflation of the cuff distal from the cuff site and the BP measurement site. The second sensor 40 may be a PPG sensor, e.g. a contact PPG sensor (such as a pulse oximetry sensor comprising one or more LEDs emitting light in the visible and/or infrared range, e.g. red and infrared light) or a remote PPG sensor (such as a camera or photo detector, as e.g. known from Verkruysse et al., "Remote plethysmographic imaging using ambient light", Optics Express, 16(26), 22 December 2008, pp. 21434-21445). PPG generally refers to the optical measurement of volume changes of an organ or a body part and in particular to the detection of volume changes due to a cardio-vascular pulse wave traveling through the body of a subject with every heartbeat. Radiation reflected by or transmitted through a skin region of the subject can be detected. The second sensor site may e.g. be the subject's hand or finger at which a contact PPG sensor may be attached, or which may be monitored by a remote PPG sensor. The second sensor site is generally a site peripheral to the first sensor site. For instance, if the cuff is attached to the upper left arm, the second sensor site may be the left hand or a finger of the left hand to which the second sensor is attached, or which is monitored by the second sensor.

The first and second sensor signals are generally acquired and sampled synchronously. Other signals than ECG and PPG signals that are related to the subject's heart rate and allow the computation of PAT and/or PTT may be used in addition or as alternative, such as a sensor detecting heart sound, a sensor detecting chest vibrations, a sensor detecting bio-impedance changes, etc.

The system 1 further comprises a device 50 as disclosed herein and as described in the following for computing a calibration value for calibrating a BP surrogate from the time-dependent BP values and the first and second time-dependent sensor signals. Further, the device 50 may optionally be configured to determine and monitor BP of the subject by use of the BP surrogate.

The system 1 may further optionally comprise an output interface 60 configured to issue any determined information, such as BP values of the subject determined and monitored by use of the BP surrogate. The output interface 60 may generally be any means that outputs information in visual or audible form, e.g. in text form, as image or diagram, as sound or spoken words, etc. For instance, the output interface 60 may be a display, a loudspeaker, a touchscreen, a computer monitor, the screen of a smartphone or tablet, etc.

The system 1 may further optionally comprise a control unit 70 that is configured to control the pressure-delivery system 10, e.g. based on a control signal provided by the device 50 or computed by the system 1. The control unit 70 may be part of the device 50 or may be an external entity.

Fig. 4 shows a diagram of an embodiment of a device 50 for calibrating a BP surrogate for use in monitoring a subject's blood pressure according to the present invention.

The device 50 comprises a BP input 51 configured to obtain time-dependent BP values during inflation of a cuff 11 of a pressure-delivery system 10 attached at a cuff site to a subject's extremity, wherein the time-dependent BP values are measured at a BP measurement site of the subject's extremity distal from the cuff site. The device 50 further comprises a sensor input 52 configured to obtain a first time-dependent sensor signal and a second time-dependent sensor signal, both related to the subject's heartbeat and measured during the inflation of the cuff, the first time-dependent sensor signal measured at a first sensor site of the subject's body and the second time-dependent sensor signal measured at a second sensor site of the subject's extremity distal from the cuff site and the BP measurement site. The BP input 51 and the sensor input 52 may be directly coupled or connected to the cuff 11 and the first and second sensors 30, 40 or may obtain (i.e. retrieve or receive) these signals from a storage, buffer, network, or bus, etc. The inputs 51 and 52 may thus e.g. be (wired or wireless) communication interfaces or data interfaces, such as a Bluetooth interface, WiFi interface, LAN interface, HDMI interface, direct cable connect, or any other suitable interface allowing signal transfer to the device 50.

The device 50 further comprises a processing unit 53. The processing unit 53 may be any kind of means configured to process the signals, generate a control signal for controlling the pressure-delivery system 10 and/or determine calibration parameters for calibration of the BP surrogate. Further, it may be configured to determine and monitor BP of the subject by use of the BP surrogate. It may be implemented in software and/or hardware, e.g. as a programmed processor or computer or app on a user device such as a smartphone, smartwatch, tablet, laptop, PC, workstation, etc.

The device 50 may further comprise an output 54 configured to output any determined information. The output 54 may generally be any interface that provides the determined information, e.g. transmits it to another device or provides it for retrieval by another device (e.g. a smartphone, computer, tablet, etc.). It may thus generally be any (wired or wireless) communication or data interface.

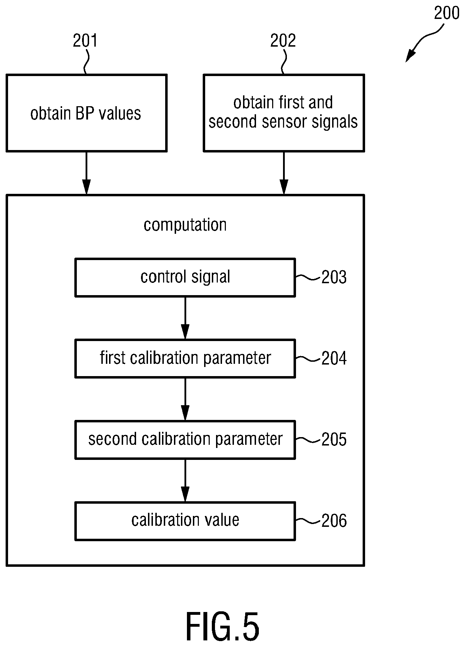

Fig. 5 shows a diagram of an embodiment of a method 200 according to the present invention. The steps of the method 200 may be carried out by the device 50, wherein the main steps of the method are carried out by the processing unit 53. The method may be implemented as computer program running on a computer or processor.

In a first step 201, time-dependent BP values during inflation of the cuff of the pressure-delivery system attached at a cuff site to a subject's extremity are obtained, wherein the time-dependent BP values are measured at a BP measurement site of the subject's extremity distal from the cuff site.

In a second step 202, a first time-dependent sensor signal and a second time-dependent sensor signal are obtained, both related to the subject's heartbeat and measured during the inflation of the cuff, the first time-dependent sensor signal measured at a first sensor site of the subject's body and the second time-dependent sensor signal measured at a second sensor site of the subject's extremity distal from the cuff site and the BP measurement site. It shall be noted that the order of the steps 201 to 202 does not indicate a chronological order, but these steps may be carried out in any chronological order. Steps 201 and 202 are preferably carried out synchronously in order to determine a useful time difference.

In a third step 203, a control signal is computed for controlling the pressure-delivery system to inflate its cuff up to a cuff pressure that causes the brachial vein of the subject's extremity to collapse, e.g., to a cuff pressure in the range equal to or larger than 25 mmHg, in particular larger than 30 mmHg.

In a fourth step 204, a first calibration parameter is computed as a ratio between a maximum MAP change and a maximum PTT change. In a fifth step 205, a second calibration parameter is computed as the absolute value of pulse arrival time, PAT, derived from the first and second time-dependent sensor signals measured after the cuff inflation. In a sixth step 206, a calibration value is computed for calibrating a BP surrogate from the first and second calibration parameters. PAT and PTT values may be extracted via appropriate feature extraction techniques from the first and second signals (e.g. ECG and PPG signals). Other features may also be derived from the single signals such as heart rate, PPG morphology features, etc.

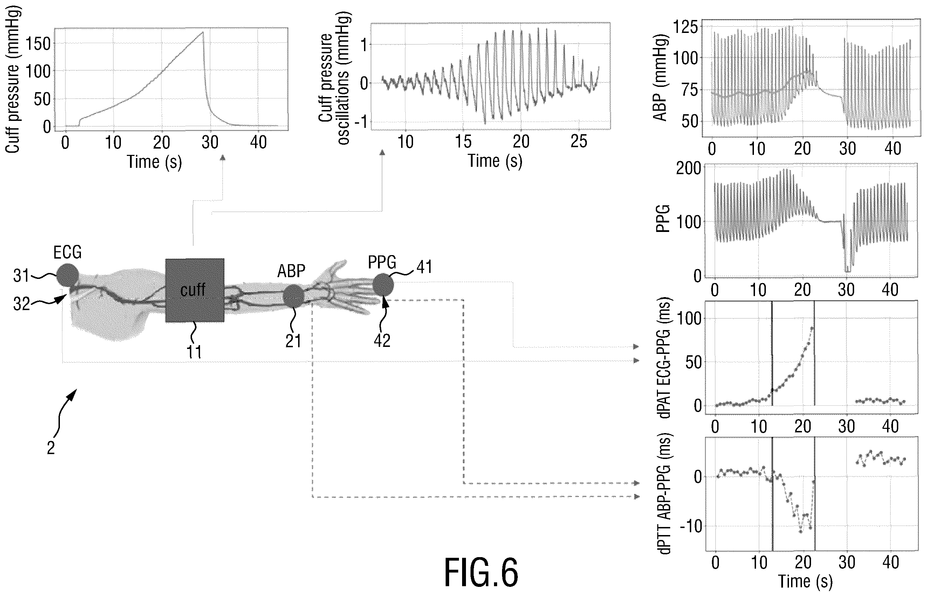

Fig. 6 shows an exemplary implementation of a system 2 according to the present invention. It comprises an ECG sensor 31 (as embodiment of a first sensor 30 configured to be attached to a first sensor site 32), a PPG sensor 41 (as embodiment of a second sensor 40 configured to be attached to a second sensor site 42), a cuff 11 and an ABP sensor 21 (as embodiment of a pressure sensor 20) to simultaneously record ECG signals, PPG signal, cuff pressure signal and an ABP signal. Exemplary signals are shown in Fig. 6 as well. PPG sensor 41, ABP sensor 21 and cuff 11 are placed at the same arm.

Fig. 6 illustrates observed changes in recorded signals during cuff inflation. As cuff pressure increases beyond systemic venous pressure (e.g. ~30 mmHg), the vein collapses and flow out of the limb is stopped; build-up of blood volume begins to occur in the limb via the artery while the cuff pressure is below internal arterial pressure. With every beat, gradually more volume of blood accumulates in the distal vasculature, as a result, distal BP increases (in the example of Fig. 6, a MAP increase of 20 mmHg is observed), distal arterial compliance decreases, distal PWV increases and distal PTT decreases (in the example of Fig. 6, a distal PTT decrease of ~10 ms is observed). A number of features has been investigated, which may be informative of the PAT-BP calibration and may allow to track BP changes via PAT as measured via ECG-PPG combination.

In an embodiment two features may be used as index of the BP - $PAT_{ECG\text{-}PPG}$ calibration. Feature 1 is

$$Slope_{distal} = \frac{max(dMAP_{Distal})}{max(dPTT_{Distal})},$$

, where $dMAP_{Distal}$ and $dPTT_{Distal}$ are measured during a cuff inflation. As explained below, the distal PTT response (as measured via ABP-PPG) to a distal BP increase (as induced via venous occlusion) reflects the 'global' PTT response (as measured via ECG-PPG) to a systemic BP change caused due to hemodynamic changes. Feature 2 is the absolute value of PAT. While Feature 2 is not related to vasculature response to cuff perturbation, it was found to correlate well with the BP - $PAT_{ECG\text{-}PPG}$ calibration. It can therefore be used to complement Feature 1 in situations of increased measurement noise. The arterial volume-Ptm and respectively the PWV-Ptm relationship is patient specific and can change due to medication/hemodynamic state. However, smaller PAT values are more likely to occur at high transmural pressure regions, where the artery is stiff and the slope dBP/dPAT is expected to be larger for a given BP change (as e.g. shown Figs. 1 and 2) when compared to lower transmural pressures (where the PAT is larger, the artery is more compliant and the dBP/dPAT slope is small). Therefore, Feature 2 is not a direct measurement of calibration (the relationship between a change in BP and a corresponding change in PAT, Eq. (1)), but can act as an indicator, a likelihood index of the calibration value, especially in situations where measurement of Feature 1 is affected by noise.

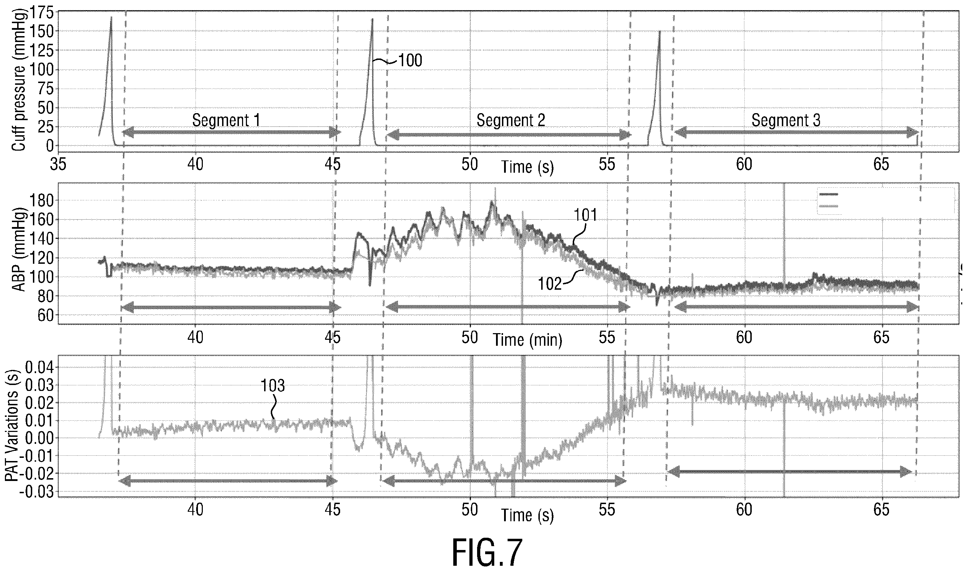

In the following feature values and their correlation to the calibration range will be explained. Fig. 7 shows a diagram illustrating a cuff pressure signal 100, a measured ABP signal 101 (measured systolic BP), an estimated BP signal 102 (estimation based on PAT) and a PAT signal 103 showing PAT variations. The signals are depicted over several segments, wherein a segment is defined as the duration between two consecutive cuff inflations. In the example shown in Fig. 7, across Segment 2, a decrease of ~30 ms in PAT corresponds to an increase in Psys of ~60 mmHg. Therefore, the ground truth calibration (Eq. (1)) of Segment 2 is ~ -2 mmHg/ms.

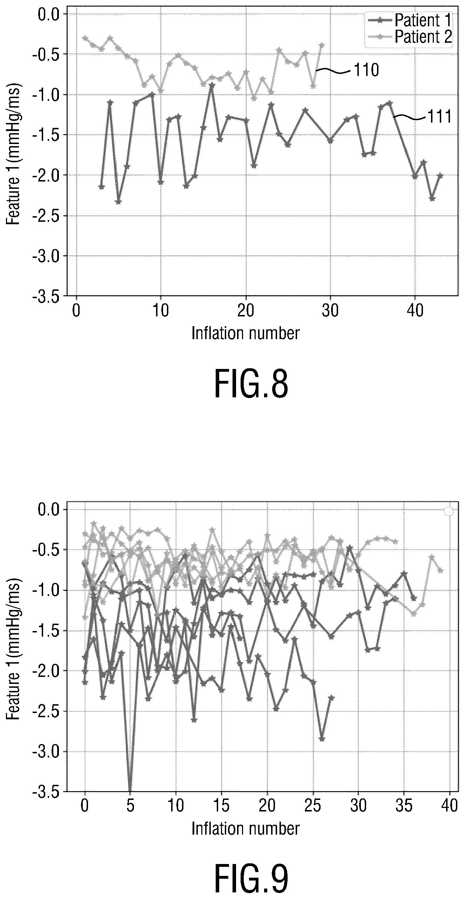

For illustration purposes, Feature 1 and Feature 2 values are depicted for two example patients (Patient 1 and Patient 2). Fig. 8 shows, per inflation number, a diagram of Feature 1 values corresponding to Patient 1 (signal 110) and Patient 2 (signal 111) as calculated during each cuff inflation. The ground-truth calibration of Patient 1 is about ~ -1.8 mmHg/ms throughout the procedure. The ground-truth calibration of Patient 2 is ~ -0.8 mmHg/ms throughout the procedure. For other patients the calibration value can change significantly throughout the procedure.

In the same format, Fig. 9 shows a diagram of example Feature 1 values acquired from ten patients as measured during cuff inflation. For illustration purposes, the Feature 1 may be grouped into two ground-truth calibration value categories, a first category in which the absolute ground-truth calibration value of the respective segment is > 1 mmHg/ms and a second category in which the absolute ground-truth calibration value of the respective segment is < 1 mmHg/ms. It can be observed that there is a threshold in feature value which can indicate the calibration range most suitable for the particular monitoring segment.

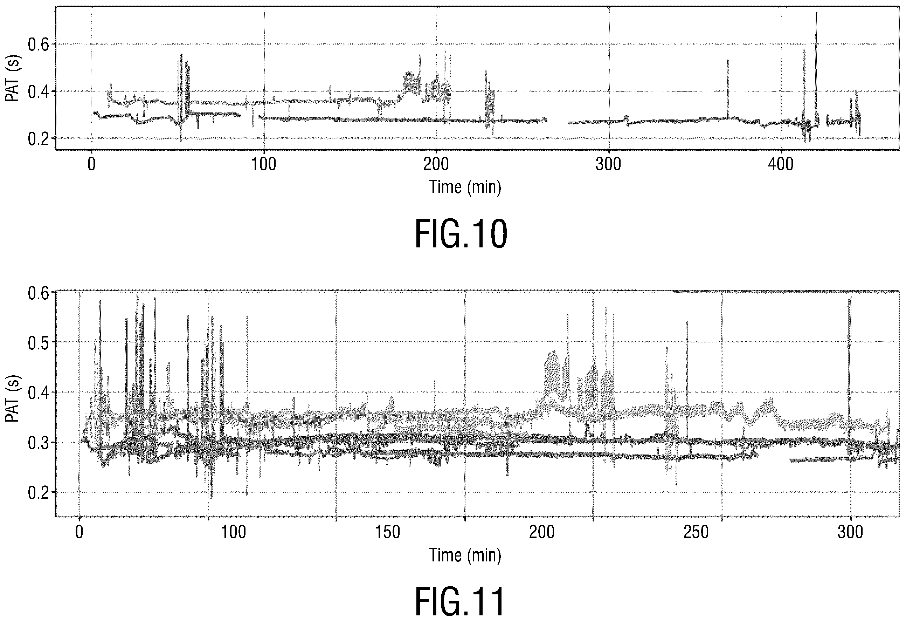

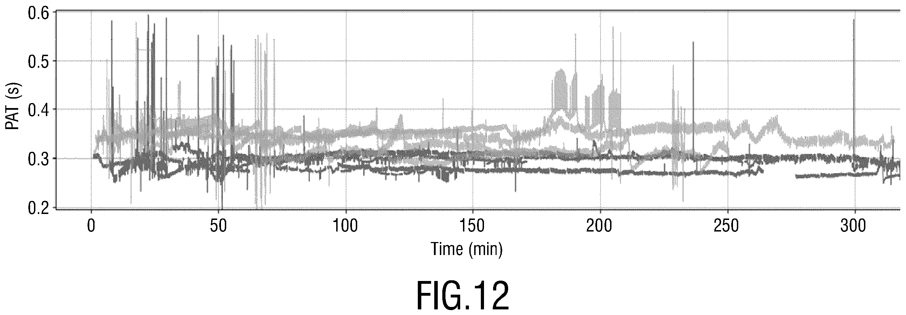

Similarly, Fig. 10 shows a diagram of Feature 2 values as measured throughout the procedure for Patient 1 and Patient 2. Figs. 11 shows a diagram of example values of Feature 2 values for nine patients, as measured throughout the procedure, which may be grouped into the two ground-truth calibration value categories mentioned above (<1 mmHg/ms and >1 mmHg/ms). Fig. 12 shows a diagram of data corresponding to a 10th patient that is added to the plot shown in Fig. 11 to illustrate an example where the feature value varies around the expected threshold throughout the procedure. In such cases of uncertainty both Feature 1 and Feature 2 values are taken into account in an embodiment.

Based on analysis across the entire database, Feature 1 threshold is ~ -0.7 mmHg/ms and Feature 2 threshold is -0.31 s. Based on this, a simple algorithm may be developed to track BP changes based on PAT. The PAT-BP calibration is obtained according to measured values of Feature 1 and Feature 2. As illustrated in Figs. 9 and 12, outliers can exist at the limit of the specified threshold values. In an embodiment, the algorithm therefore takes into account both feature values and computes a level of uncertainty in order to ensure that PAT-based BP estimation does not produce larger errors than the standard NIBP (0-hold BP estimation based on last cuff-based measurement).

An exemplary embodiment of a method to determine a calibration value will be explained in the following. For each segment, as shown in Fig. 7, the following algorithm may be followed to identify the calibration value for each segment: A first step includes to compute Feature 1 (F1) based on signals measured during the inflation occurring immediately prior to the segment. A second step includes to compute Feature 2 (F2) based on signals recorded at the beginning of the segment (beginning of the segment is defined in this case as, e.g., 30 s after the cuff pressure has dropped under, e.g., 10 mmHg). F2 is computed as the measured PAT averaged over, e.g., a 10 second duration at the beginning of the segment.

If the F1 value and the F2 value are both sufficiently different from their respective thresholds (e.g., differ by more than -5% of threshold value), the following will be determined:

- If F1 < F1_threshold and F2 < F2_threshold, then the calibration = -1.8 mmhg/ms.
- If F1 > F1_threshold and F2 > F2_threshold, then the calibration = -0.8 mmhg/ms.
- If only F1 is considerably different from its respective limit: F2 is not taken into account, the calibration is assigned either -1.8 or -0.8 mmhg/ms depending on the F1 value.

- If only F2 is considerably different from its respective limit: F1 is not taken into account, the calibration is assigned either -1.8 or -0.8 mmhg/ms depending on F2 value.
- If both feature values are close to their respective limits:

    - If mean(F 1 from previous inflations) < F1_threshold and mean(F2 from previous inflations) < F2_threshold, then the calibration = -1.8 mmhg/ms;
    - If mean(F1 from previous inflations) > F1_threshold t and mean(F2 from previous inflations) > F2_threshold, then the calibration = -0.8 mmhg/ms;

- If at this stage calibration is unassigned, the BP = BPreference based on previous NIBP measurement; PAT is not taken into account.

After the calibration value is found, systolic pressure may be estimated as follows:

$$Estimated\_Psys = calibration\_value * dPAT + Psys\_ref$$

where Psys_ref is obtained via standard NIBP during the inflation immediately prior to the segment. If no calibration has been found, due to, e.g., feature values being too close to the threshold, then Estimated_Psys = Psys_ref.

This algorithm is intended only as an example of a potential method of making use of the identified feature thresholds. The algorithm can be made more complex or less complex, different weights may be given to the features, the choice of taking into account multiple previous inflations may be removed or may be given more weight in the decision tree, etc., i.e., multiple variations are generally possible.

The performance of the feature-based algorithm has been compared with the performance of the standard intermittent NIBP. It has been found that the algorithm achieves significantly improved performance in detection of BP changes when compared to standard NIBP. The performance evaluation may be conducted as described below. For each segment, the maximum error and root mean square error (RMSE) are calculated for PAT-based BP estimation (feature-based) and standard NIBP (0-hold of the systolic value obtained during the cuff inflation performed immediately prior to the segment).

An implementation of the measurement principle may use two discrete standardized slope values. Improvements in PAT-based BP estimation are already achieved at this level of granularity. Simply identifying whether the calibration value is large or small results in improved BP tracking when compared to standard NIBP. Potential further improvements may be achieved by mapping the feature values to better defined, or larger number of calibration value categories.

Further, a BP change may be induced in the distal arm via a cuff; the resulting change in distal PTT can be interpreted to obtain the PAT-BP calibration. In other words, the distal PTT response to cuff-induced BP change is consistent with the 'global' PTT response (as measured via ECG-PPG) to BP changes occurring as result of hemodynamic regulation.

Embodiments of the present invention can be employed to non-invasively assess the BP-PTT interaction in the distal arm during cuff inflation. An index of the BP change magnitude and an index of the PTT change magnitude may be needed. An index for the increase in distal BP may be obtained via the PPG signal response to cuff inflation.

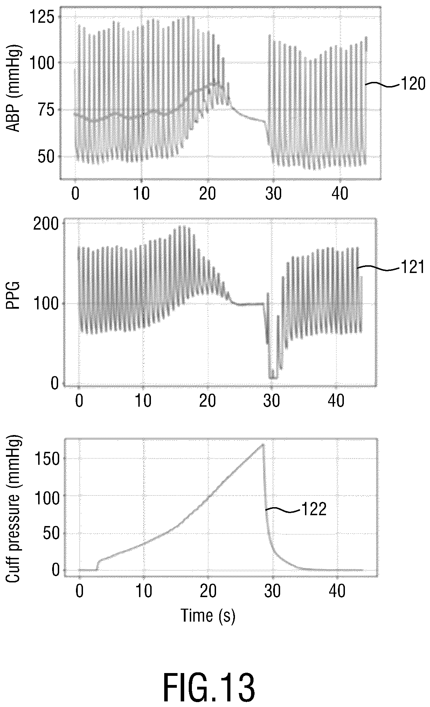

Fig. 13 shows a diagram of an invasive arterial line signal 120, a PPG signal 121 and a cuff pressure signal 121 acquired during cuff inflation (e.g., via the setup illustrated in Fig. 6). These signals follow similar patterns. For example, the increase in the lower envelope of the PPG signal likely correlates with the magnitude of the distal BP increase. Also, the slope of the lower PPG signal envelope likely contains information related to the change in BP.

As an alternative, a hemodynamic model may be used to link duration of venous occlusion with expected delta volume of blood present in the limb. Under certain assumptions (e.g., a linear increase in distal blood volume with each heartbeat throughout venous occlusion, a linear increase in BP as result to a linear increase in blood volume), the resulting PPG and PTT measurement, HR, duration of venous occlusion can be inputted towards a hemodynamic model which outputs an approximation of the BP increase. Empirical findings from studies involving invasive measurements may also be used as parameters of such a model. Depending on available measurements, flow acquired via Doppler ultrasound may also act as input for the model. Other model inputs can also be acquired via tonometry or volume clamp sensors.

In another embodiment, characteristics of the distal dPTT function with respect to time may be indicative of the magnitude of the BP change (how quickly maximum dPTT is reached, or other similar indexes may correlate with the BP change magnitude).

Various occlusion strategies, or combinations of occlusion strategies may be used (cuff pressure plateaus/ cuff inflation/deflation, duration of occlusion, etc.).

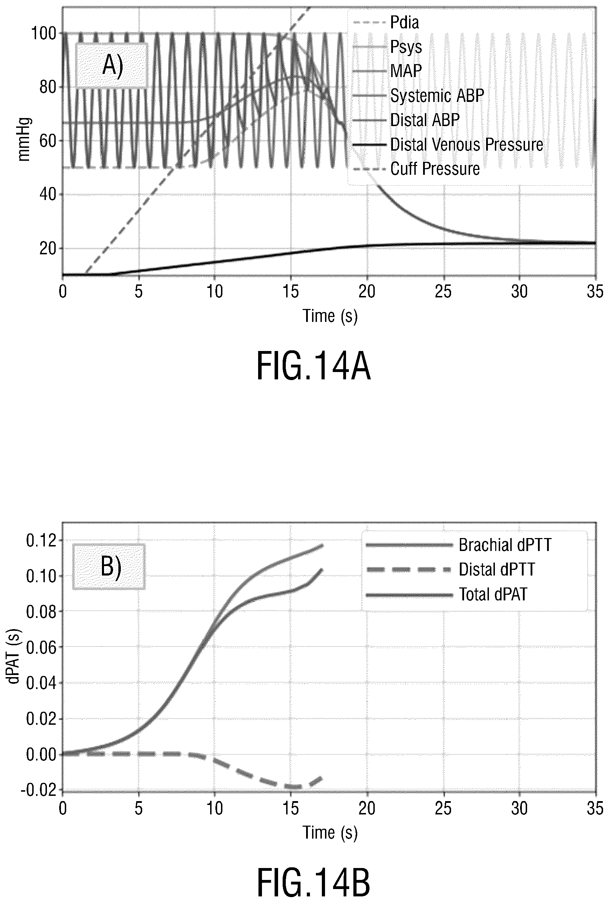

An index of the PTT change magnitude can be obtained via in-depth analysis of the measured ECG-PPG PAT signal. Fig. 14A shows a diagram illustrating a simulated systemic arterial pressure, cuff pressure and resulting distal arterial and venous pressure. Fig. 14B shows a diagram illustrating a simulated total $\varDelta$PAT, $\varDelta PTT_{brachial}$, and $\varDelta PTT_{distal}$.

As illustrated via the simulation illustrated in Fig. 14, the dPAT signal (Fig. 14B) incorporates changes occurring in PTT along proximal, brachial, as well as distal segments of the artery. No change in PTT is expected proximally from the cuff, an increase in PTT is expected over the brachial segment. Therefore, the magnitude of the identified decrease in dPAT can be attributed to changes in PTT occurring distally from the cuff. In this way, an approximation, or an index of the decrease in distal PTT can be obtained.

Alternatively, for increased accuracy, ultrasound may be used, e.g., Doppler signal as measured via wearable ultrasound patch in combination with the PPG signal can be used to estimate distal PTT.

The solution of the present invention aims to solve specifically for the issue of calibration (an x ms change in PAT corresponds to a y mmHg change in BP, Eq. (1)). There are other factors possibly affecting PAT-based BP estimation, for example:

- a change in PEP halfway through a segment, arrhythmia;
- motion artifacts due to medical procedure;
- noisy signals due to sensor placement, procedure, difficult to identify signal fiducial points;
- change in signal baseline, e.g. due to movement of arm, or re-adjustment of PPG sensor;
- segments where particularly dynamic effects take place, e.g., very severe hemodynamic changes where calibration is not constant throughout one segment; such unstable hemodynamic status however may necessitate invasive measurements.

In such cases, no calibration value will lead to correct estimation of BP. Solutions in these cases may include algorithms to identify quality of the recorded signals, increased attention in placement of sensors, limit motion artifacts, careful selection of patient groups (non-cardiac, no endothelial dysfunction), etc.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

It is to be understood that any of the previous steps described in relation to embodiments and/or training steps described above can be performed by a specific-purpose computer system or general-purpose computer system, or a computer-readable medium, or data carrier system configured to carry out any of the steps described previously. The computer system can include a set of software instructions that can be executed to cause the computer system to perform any of the methods or computer-based functions disclosed herein. The computer system may operate as a standalone device or may be connected, for example using a network, to other computer systems or peripheral devices. In embodiments, a computer system performs logical processing based on digital signals received via an analogue-to-digital converter.

Some portions of the description are presented in terms of symbolic representations of operations on non-transient signals stored within a computer memory. These descriptions and representations are used by those skilled in the data processing arts to convey the substance of their work most effectively to others skilled in the art. Such operations typically require physical manipulations of physical quantities. Usually, though not necessarily, these quantities take the form of electrical, magnetic, or optical signals capable of being stored, transferred, combined, compared, and otherwise manipulated. It is convenient at times, principally for reasons of common usage, to refer to these signals as bits, values, elements, symbols, characters, terms, numbers, or the like. Furthermore, it is also convenient at times to refer to certain arrangements of steps requiring physical manipulation of physical quantities as modules or code devices, without loss of generality.

The computer system further includes a main memory and a static memory, where memories in the computer system communicate with each other and the processor via a bus. Either or both main memory and the static memory may be considered representative examples of the memory of the controller, and store instructions used to implement some, or all aspects of methods and processes described herein. Memories described herein are tangible storage mediums for storing data and executable software instructions and are non-transitory during the time software instructions are stored therein. The main memory and the static memory are articles of manufacture and/or machine components. The main memory and the static memory are computer-readable mediums from which data and executable software instructions can be read by a computer (or e.g., the processor). Each of the main memory and the static memory may be implemented as one or more of random access memory (RAM), read only memory (ROM), flash memory, electrically programmable read only memory (EPROM), electrically erasable programmable read-only memory (EEPROM), registers, a hard disk, a removable disk, tape, compact disk read only memory (CD-ROM (Compact Disk - Read Only Memory)), digital versatile disk (DVD), floppy disk, Blu-ray disk, or any other form of storage medium known in the art. The memories may be volatile or non-volatile, secure and/or encrypted, unsecure and/or unencrypted.

"Memory" is an example of a computer-readable storage medium. Computer memory is any memory which is directly accessible to a processor. Examples of computer memory include RAM memory, registers, and register files. References to "computer memory" or "memory" should be interpreted as possibly being multiple memories. The memory may for instance be multiple memories within the same computer system. The memory may also be multiple memories distributed amongst multiple computer systems or computing devices. The memory may store various software applications including computer executable instructions, that when run on the processor, implement the methods and systems set out herein. Other forms of memory, such as a storage device and a mass storage device, may also be included and accessible by the processor (or processors) via the bus. The storage device and mass storage device can each contain any or all of the methods and systems discussed herein.

In accordance with various embodiments of the present disclosure, the methods described herein may be implemented using a hardware computer system that executes software programs. Further, in an exemplary, non-limited embodiment, implementations can include distributed processing, component/object distributed processing, and parallel processing. Virtual computer system processing may implement one or more of the methods or functionalities as described herein, and a processor described herein may be used to support a virtual processing environment.

The illustrations of the embodiments described herein are intended to provide a general understanding of the structure of the various embodiments. The illustrations are not intended to fully describe all the elements and features of the disclosure described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

## Claims

1. Device for calibrating a blood pressure, BP, surrogate for use in monitoring a subject's blood pressure, the device comprising:

   a BP input (51) configured to obtain time-dependent BP values during inflation of a cuff (11) of a pressure-delivery system (10) attached at a cuff site to a subject's extremity, wherein the time-dependent BP values are measured at a BP measurement site of the subject's extremity distal from the cuff site;
   a sensor input (52) configured to obtain a first time-dependent sensor signal and a second time-dependent sensor signal, both related to the subject's heartbeat and measured during the inflation of the cuff, the first time-dependent sensor signal measured at a first sensor site of the subject's body and the second time-dependent sensor signal measured at a second sensor site of the subject's extremity distal from the cuff site and the BP measurement site; and
   a processing unit (53) configured to compute

   - a control signal for controlling the pressure-delivery system (10) to inflate its cuff up to a cuff pressure that causes the brachial vein of the subject's extremity to collapse;
   - a first calibration parameter as a ratio between a maximum mean arterial pressure, MAP, change and a maximum PTT change;
   - a second calibration parameter as the absolute value of pulse arrival time, PAT, derived from the first and second time-dependent sensor signals measured after the cuff inflation; and
   - a calibration value for calibrating a BP surrogate from the first and second calibration parameters.

2. Device according to claim 1,
   wherein the processor is configured to compute the maximum MAP change in distal mean arterial pressure, MAP, as a difference between a maximum MAP value during the cuff inflation and the MAP value at the beginning of the cuff inflation, the MAP values being obtained from the obtained time-dependent BP values.

3. Device according to claim 1 or 2,
wherein the processor is configured to compute the maximum PTT change in distal pulse transit time, PTT, as a time interval between a pulse detected at the BP measurement site and the pulse detected at the second sensor site.

4. Device according to any one of the preceding claims,
wherein the processor is configured to compute the calibration value by setting the calibration value to a first calibration value if:

- the first calibration parameter is smaller than and outside a first parameter range from a first parameter threshold and the second calibration parameter is smaller than and outside a second parameter range from a second parameter threshold; or
- the second calibration parameter is within the second parameter range from the second parameter threshold and the first calibration parameter is smaller than and outside the first parameter range from the first parameter threshold; or
- the first calibration parameter is within the first parameter range from the first parameter threshold and the second calibration parameter is smaller than and outside the second parameter range from the parameter threshold; or
- the first calibration parameter is within the first parameter range from the first parameter threshold, the second calibration parameter is within the second parameter range from the second parameter threshold, a mean of a plurality of last first calibration parameters is smaller than the first parameter threshold and a mean of a plurality of last second calibration parameters is smaller than the second parameter threshold.

5. Device according to any one of the preceding claims,
wherein the processor is configured to compute the calibration value by setting the calibration value to a second calibration value if:

- the first calibration parameter is larger than and outside a first parameter range from a first parameter threshold and the second calibration parameter is larger than and outside a second parameter range from a second parameter threshold; or
- the second calibration parameter is within the second parameter range from the second parameter threshold and the first calibration parameter is larger than and outside the first parameter range from the first parameter threshold; or
- the first calibration parameter is within the first parameter range from the first parameter threshold and the second calibration parameter is larger than and outside the second parameter range from the parameter threshold; or
- the first calibration parameter is within the first parameter range from the first parameter threshold, the second calibration parameter is within the second parameter range from the second parameter threshold, a mean of a plurality of last first calibration parameters is larger than the first parameter threshold and a mean of a plurality of last second calibration parameters is larger than the second parameter threshold.

6. Device according to claim 4 and/or 5,
wherein one or more of the first calibration value, the second calibration value, the first parameter threshold, the second parameter threshold, the first parameter range and the second parameter range are predetermined values.

7. Device according to any one of claims 4 to 6, wherein

the first calibration value is in the range of -1.5 to -2.5 mmHg/ms, in particular a value of -1.8 mmHg/ms $\pm$ 10%, and/or
the second calibration value is in the range of -1 to -0.5 mmHg/ms, in particular a value of -0.8 mmHg/ms $\pm$ 10%, and/or
the first parameter threshold is in the range of -1 to -0.5 mmHg/ms, in particular a value of -0.7 mmHg/ms $\pm$ 10%, and/or
the second parameter threshold is in the range of -0.4 to -0.2 mmHg/ms, in particular a value of -0.31 mmHg/ms $\pm$ 10%, and/or
the first parameter range is in the range of 2.5 % to 7.5 %, in particular a value of 5 %, and/or

the second parameter range is in the range of 2.5 % to 7.5 %, in particular a value of 5 %.

8. Device according to any one of the preceding claims,
wherein the processor is configured to compute a control signal for controlling the pressure-delivery system (10) to inflate its cuff up to a cuff pressure equal to or larger than 25 mmHg, in particular larger than 30 mmHg or in the range of 25 to 35 mmHg.

9. Device as claimed in any one of the preceding claims,
wherein the first time-dependent sensor signal is an ECG signal and/or the second time-dependent sensor signal is a photoplethysmography, PPG, signal, in particular a contact PPG signal or a remote PPG signal.

10. Device as claimed in any one of the preceding claims,
wherein the processing unit (53) is configured to determine BP values, in particular systolic BP values, by use of the computed calibration value and the first and second time-dependent sensor signals measured when no pressure is delivered to the subject's extremity.

11. Device as claimed in claim 10,
wherein the processing unit (53) is configured to determine a BP value, in particular a systolic BP value, by multiplying computed calibration value with an absolute PAT value derived from the first and second time-dependent sensor signals and adding the result of the multiplication to a reference BP value, in particular a reference systolic BP value.

12. System for calibrating a blood pressure, BP, surrogate for use in monitoring a subject's blood pressure, the system comprising:

   a pressure-delivery system (10) comprising a cuff (11) configured to be attached at a cuff site to a subject's extremity and to deliver a pressure to the subject's extremity by inflating the cuff;
   a BP pressure sensor (20) configured to acquire time-dependent BP values at a BP measurement site of the subject's extremity distal from the cuff site during inflation of the cuff up to a cuff pressure that causes the brachial vein of the subject's extremity to collapse;
   a first sensor (30) configured be attached to a first sensor site of the subject's body and to acquire a first time-dependent sensor signal related to the subject's heartbeat during the inflation of the cuff;
   a second sensor (40) configured be attached to a second sensor site of the subject's extremity and to acquire a second time-dependent sensor signal related to the subject's heartbeat during the inflation of the cuff distal from the cuff site and the BP measurement site; and
   a device (50) as claimed in any one of the preceding claims for computing a calibration value for calibrating a BP surrogate from the time-dependent BP values and the first and second time-dependent sensor signals.

13. Method for calibrating a blood pressure, BP, surrogate for use in monitoring a subject's blood pressure, the method comprising:

   - obtaining time-dependent BP values during inflation of a cuff (11) of a pressure-delivery system (10) attached at a cuff site to a subject's extremity, wherein the time-dependent BP values are measured at a BP measurement site of the subject's extremity distal from the cuff site;
   - obtaining a first time-dependent sensor signal and a second time-dependent sensor signal, both related to the subject's heartbeat and measured during the inflation of the cuff, the first time-dependent sensor signal measured at a first sensor site of the subject's body and the second time-dependent sensor signal measured at a second sensor site of the subject's extremity distal from the cuff site and the BP measurement site; and
   - computing a control signal for controlling the pressure-delivery system (10) to inflate its cuff up to a cuff pressure that causes the brachial vein of the subject's extremity to collapse;
   - computing a first calibration parameter as a ratio between a maximum mean arterial pressure, MAP, change and a maximum PTT change;
   - computing a second calibration parameter as the absolute value of pulse arrival time, PAT, derived from the first and second time-dependent sensor signals measured after the cuff inflation; and
   - computing a calibration value for calibrating a BP surrogate from the first and second calibration parameters.

14. Computer program comprising program code means for causing a computer to carry out the steps of the method as claimed in claim 13 when said computer program is carried out on the computer.

FIG.1A

FIG.1B

FIG.2B

FIG.2D

FIG.2A

FIG.2C

1

pressure - delivery
system

cuff

10

BP sensor

20

control unit

70

device

50

output
interface

60

first sensor
(e.g. ECG)

30

second sensor
(e.g. PPG)

40

# FIG.3

50

BP
input

51

sensor
input

52

processing
unit

53

output

54

# FIG.4

200

| 201 | 202 |
|---|---|
| obtain BP values | obtain first and second sensor signals |

computation

control signal ⌇203

↓

first calibration parameter ⌇204

↓

second calibration parameter ⌇205

↓

calibration value ⌇206

# FIG.5

FIG.6

FIG.7

FIG.8

FIG.9

FIG.10

FIG.11

FIG.12

FIG.13

FIG.14A

FIG.14B

Europäisches
Patentamt

European
Patent Office

Office européen
des brevets

**EUROPEAN SEARCH REPORT**

**Application Number**

EP 24 17 0498

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LOUREIRO JOÃO ET AL: "Towards continuous non-invasive blood pressure measurements-interpretation of the vasculature response to cuff inflation", FRONTIERS IN PHYSIOLOGY, vol. 14, 2 June 2023 (2023-06-02), XP093171955, CH ISSN: 1664-042X, DOI: 10.3389/fphys.2023.1172688 * the whole document * | 1-14 | INV. A61B5/021 A61B5/022 A61B5/0225 |
| A | US 2014/276145 A1 (BANET MATT [US] ET AL) 18 September 2014 (2014-09-18) * equation 10; paragraphs [0097], [0151] * | 1-14 | |
| A | CATTIVELLI F S ET AL: "Noninvasive Cuffless Estimation of Blood Pressure from Pulse Arrival Time and Heart Rate with Adaptive Calibration", WEARABLE AND IMPLANTABLE BODY SENSOR NETWORKS, 2009. BSN 2009. SIXTH INTERNATIONAL WORKSHOP ON, IEEE, PISCATAWAY, NJ, USA, 3 June 2009 (2009-06-03), pages 114-119, XP031522342, ISBN: 978-0-7695-3644-6 * page 116, column 1, paragraph 2 * * page 118, column 1, paragraph 3 * | 1-14 | TECHNICAL FIELDS SEARCHED (IPC) A61B |

-/--

The present search report has been drawn up for all claims

| Place of search | Date of completion of the search | Examiner |
|---|---|---|
| The Hague | 13 June 2024 | Athanasiadis, I |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

**Application Number**

EP 24 17 0498

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SHAOXIONG SUN ET AL: "Systolic blood pressure estimation using ECG and PPG in patients undergoing surgery", ARXIV.ORG, CORNELL UNIVERSITY LIBRARY, 201 OLIN LIBRARY CORNELL UNIVERSITY ITHACA, NY 14853, 23 December 2021 (2021-12-23), XP091147200, * the whole document * | 1-14 | |

-----

TECHNICAL FIELDS
SEARCHED      (IPC)

The present search report has been drawn up for all claims

| Place of search | Date of completion of the search | Examiner |
|---|---|---|
| The Hague | 13 June 2024 | Athanasiadis, I |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

............................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 24 17 0498

This annex lists the patent family members relating to the patent documents cited in  the above-mentioned European search report.
The members are as contained in the European Patent Office EDP file on
The European Patent Office is in no way liable for these particulars which are merely given for the purpose of information.

13-06-2024

| Patent document cited in search report | Publication date | Patent family member(s) | Publication date |
|---|---|---|---|
| US 2014276145 A1 | 18-09-2014 | EP 2162059 A1 | 17-03-2010 |
| | | US 2009018453 A1 | 15-01-2009 |
| | | US 2010160794 A1 | 24-06-2010 |
| | | US 2010160795 A1 | 24-06-2010 |
| | | US 2010160796 A1 | 24-06-2010 |
| | | US 2010160797 A1 | 24-06-2010 |
| | | US 2010168589 A1 | 01-07-2010 |
| | | US 2014276145 A1 | 18-09-2014 |
| | | US 2018055389 A1 | 01-03-2018 |
| | | WO 2008154643 A1 | 18-12-2008 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2023072842 A1
- WO 2023072730 A1
- US 11317819 B2

**Non-patent literature cited in the description**

- **VERKRUYSSE et al.** Remote plethysmographic imaging using ambient light. *Optics Express*, 22 December 2008, vol. 16 (26), 21434-21445